# EUROPEAN PATENT SPECIFICATION

(11) **EP 2 957 230 B1**
(45) Date of publication and mention of the grant of the patent: **20.12.2017**
(21) Application number: 15170780.9
(22) Date of filing: 05.06.2015
(51) Int. Cl.: A61B 6/04, A61G 13/02

(54) **REMOTE CONTROL FOR PATIENT SUPPORT SYSTEM**
FERNBEDIENUNG FÜR PATIENTENLAGERUNGSSYSTEM
TÉLÉCOMMANDE POUR SYSTÈME DE SUPPORT DE PATIENT

(30) Priority: 20.06.2014 GB 201411011
(43) Date of publication of application: 23.12.2015
(73) Proprietor: Elekta Limited, West Sussex RH10 9RR (GB)
(72) Inventor: Truta, Mihai, Crawley, Sussex RH10 9RR (GB)
(74) Representative: Hall, Christopher David

(56) References cited:
- DE-A1-102012 220 667
- DE-C1- 4 224 246
- US-A1- 2009 126 115

## Description

### Field of the Invention

This invention relates to a patient support system and, in particular, to a means for manipulating the patient support system.

### Background to the Invention

A patient support may be used in a medical environment to support a patient who is undergoing examination or a medical procedure, for example a radiotherapy procedure.

A known patient support includes an upper surface upon which a patient can sit or lie while a procedure is carried out. The upper surface may be mounted upon a base and may include functionality to enable the upper surface of the support to be moved relative to the base of the support. For example, a patient may need to be positioned so that a body portion, for example the left lung, is positioned over the centre of the base of the patient support. This may be achieved by translating the upper surface of the support to the right with respect to the base, so that the left lung of the patient is positioned centrally relative to the base of the support.

Existing control systems used for manipulating the position of a bed of a patient support or manipulating the position of the entire patient support can be complicated and the use of such control systems is often not intuitive. Thus, it can be difficult to move the patient into the required position.

US 2009/0126115 discloses a remote controller for an adjustable support apparatus for supporting a patient. An input device is configured to accept control commands for adjusting the support apparatus.

DE 10 2012 220 667 discloses an operating unit for a medical apparatus, the operating unit configured for attachment to different sides of a driven movable element of a medical apparatus. The operating unit is adapted to control movement of the upper surface of a patient support and defines a longitudinal axis. When the operating unit is oriented such that its longitudinal axis is parallel to a first or second movement axis of the patient support, the upper surface is moveable along the first or second axis. DE 42 24 246 discloses a manual control knob for controlling a position of a patient table.

### Summary of Invention

A first aspect of the invention provides a patient support system, comprising a patient support having a base and an upper surface, the upper surface moveable relative to the base in a direction along at least one of a first axis, a second axis orthogonal to the first axis, and a third axis orthogonal to the first and second axes; and a control unit for controlling movement of the upper surface, the control unit defining a longitudinal axis; wherein the direction of movement of the upper surface relative to the base is determined by the orientation of the longitudinal axis of the control unit relative to the patient support.

When the control unit is oriented such that its longitudinal axis is parallel to or within a defined angular range of the first axis, the upper surface is moveable along the first axis.

When the control unit is oriented such that its longitudinal axis is parallel to or within a defined angular range of the second axis, the upper surface is moveable along the second axis. When the control unit is oriented such that its longitudinal axis is parallel to or within a defined angular range of the third axis, the upper surface is moveable along the third axis.

When the control unit is oriented such that its longitudinal axis is outside the defined angular ranges, control of the movement of the upper surface by the control unit is prohibited.

The defined angular range about each axis may be a range of 30 degrees.

The control unit may comprise a first user interface for receiving a user input. The upper surface may be configured to move in response to a user input received via the first user interface.

The first user interface may comprise a sliding input device, the sliding input device moveable along a track.

The control unit may have a front end and a rear end. The sliding input device may be movable between a front end of the track and a rear end of the track, the front end and the rear end of the track corresponding to the front end and the rear end of the control unit respectively.

The sliding input device may be biased towards a central position along the track.

When the control unit is oriented such that its longitudinal axis is parallel to or within the defined angular range of a particular one of the first, second and third axes, the direction of movement of the upper surface along the particular axis may correspond to a direction of movement of the sliding input device along the track.

The upper surface may be rotationally moveable relative to the base about at least one of the first axis, the second axis, and the third axis.

The control unit may further comprise a toggle switch, moveable between a first position and a second position. When the toggle switch is in the first position, the first user interface may be configured to effect linear movement of the upper surface and, when the toggle switch is in the second position, the first user interface may be configured to effect rotational movement of the upper surface.

When the control unit is oriented such that its longitudinal axis is parallel to or within the defined angular range of the first axis, the upper surface may be rotatable about one of first, second or third axes. When the control unit is oriented such that its longitudinal axis is parallel to or within the defined angular range of the second axis, the upper surface may be rotatable about another of the first, second or third axes. When the control unit is oriented such that its longitudinal axis is parallel to or within the defined angular range of the third axis, the upper surface may be rotatable about the other of the first, second or third axes.

The sliding input device may be rotatable with respect to the control unit. The sliding input device may be configured such that rotating the sliding input device effects rotation of the upper surface relative to the base of the patient support.

The control unit may include at least one transmitter and the patient support may include at least one receiver. The at least one transmitter may be configured to communicate with the at least one receiver in order for the orientation of the control unit relative to the patient support to be determined.

The at least one transmitter may comprise an ultrasound transmitter, infrared transmitter and/or an RFID transmitter and the at least one receiver may comprise an ultrasound receiver, an infrared receiver and/or an RFID receiver. The control unit may comprise an inertial measurement device for determining one or more of the orientation and position of the control unit relative to the patient support. The inertial measurement device may comprise one or more of an accelerometer, a gyroscope, a compass and a magnetometer.
The position and orientation of the control unit may be determined by one or more external sensors. Similarly, the position and orientation of the patient support may be determined by one or more external sensors.

The control unit may comprise an activation switch moveable between a first position and a second position, and biased towards the second position. When the activation switch is in the first position, functionality of the first user interface may be enabled and, when the activation switch is in the second position, functionality of the first user interface may be prevented.

It will be appreciated that features of the various aspects of the invention may be combined with those of other aspects of the invention.

### Brief Description of the Drawings

Embodiments of the invention will now be described, strictly by way of example only, with reference to the accompanying drawings, of which:
Figure 1A is a perspective view of a patient support system;
Figure 1B is a diagram showing possible directions of movement of the patient support system of Figure 1A;
Figures 2A and 2B are perspective views of a control unit for manipulating the position of a patient support system;
Figure 3 is a schematic view of a patient support system and a control unit constructed in accordance with an embodiment of the invention;
Figure 4 is a schematic view of a patient support system and a control unit constructed in accordance with an alternative embodiment of the invention;
Figures 5, 6 and 7 are schematic views of a patient support system and a control unit constructed in accordance with various embodiments of the invention;
Figure 8A is a side view of a control unit constructed in accordance with an embodiment of the invention; and
Figure 8B is a perspective view of the control unit of Figure 8A.

### Description of the Embodiments

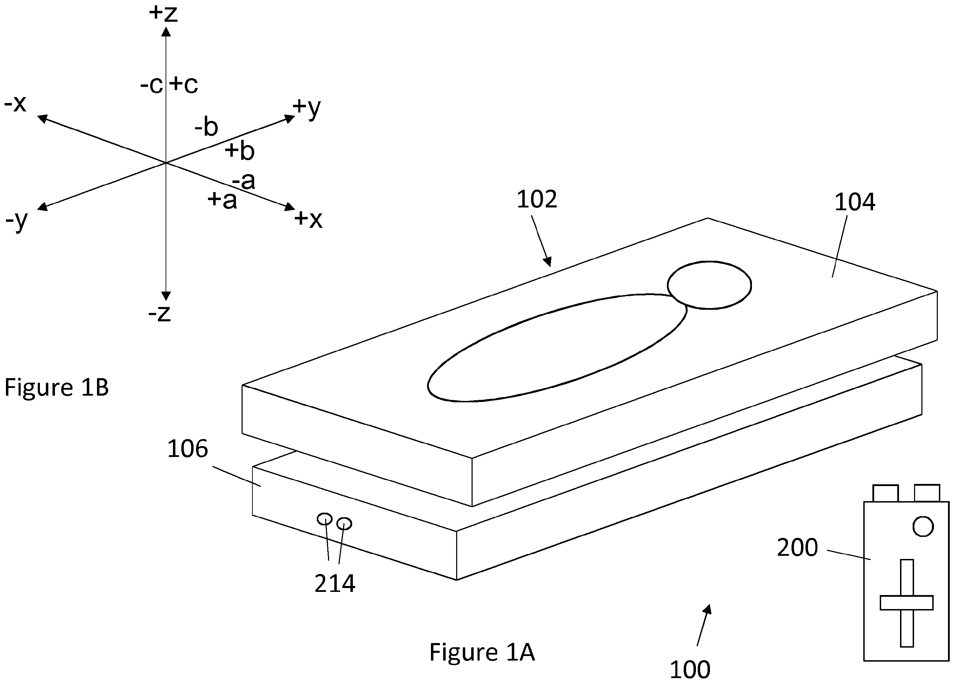

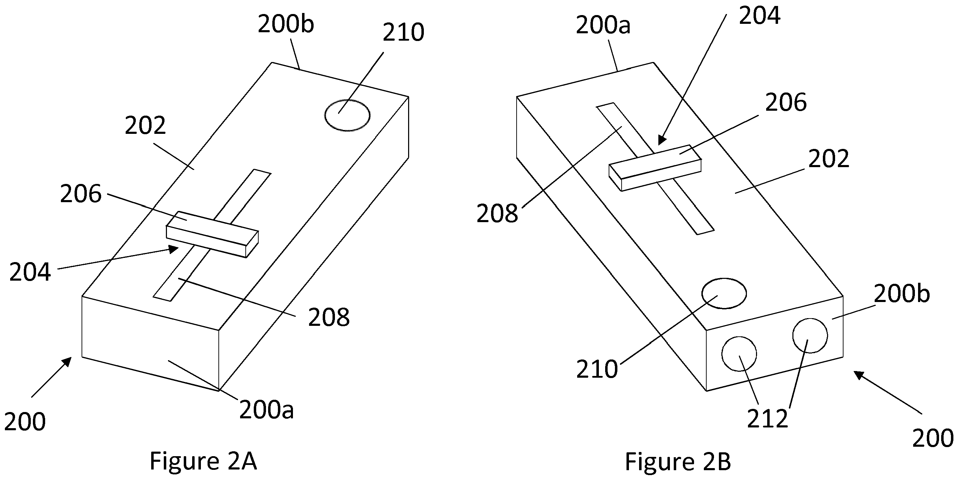

Referring to the drawings, Figure 1 shows a patient support system 100 that includes a patient support 102 having an upper surface 104 on which a patient can sit or lie down for examination or for treatment. The upper surface 104 is moveably mounted onto a base 106. The upper surface 104 may be moved relative to the base 106 by a mechanism (not shown) located between, or within one or more of, the upper surface and the base. The base 106 may also be moveable, for example along a surface, or adjustable, for example to increase or reduce the height of the upper surface 104 above the ground. Movement of the upper surface 104 relative to the base 106 is achieved using a control unit 200 which may be connected to the patient support 102 by a cable or wirelessly using a known wireless communication standard such as IEEE 802.11a, 802.11g, 802.11n, Bluetooth or RFID. The control unit 200 will be discussed in more detail with reference to Figures 2A and 2B.

Depending on the use of the patient support system, it may be necessary to accurately move the patient being examined or treated in one or more number of directions. Figure 1B shows the possible directions of movement of the upper surface 104 relative to the base 106 and the possible rotational movements of the upper surface. Movement in the +z direction represents a linear movement of the upper surface 104 upwards, away from the base 106, and movement in the -z direction represents a linear movement of the upper surface downwards, towards the base. Movement in the +y direction represents a linear movement of the upper surface 104 in that direction relative to the base 106, and movement in the -y direction represents a linear movement of the upper surface in that direction relative to the base. In this embodiment, and using this notation, it will be appreciated that movement in the +y and -y directions represent linear movement along a longitudinal axis of the patient support 102, and perpendicular to the x and z axes. Movement in the +x direction represents a linear movement of the upper surface 104 in that direction relative to the base 106, and movement in the -x direction represents a linear movement of the upper surface 104 in that direction relative to the base 106. Again, in this embodiment, and using this notation, it will be appreciated that movement in the +x and -x directions represents movement of the upper surface 104 in a direction perpendicular to the y and z axes.

The base 106 may include a mechanism (not shown) to enable it to extend or retract in the +z or -z directions, along the z axis, for example to increase its height. Similarly, movement of the upper surface 104 along the z axis may be achieved by extending or retracting the base along the z axis, while the upper surface 104 is not moved relative to the base.

In addition to linear movement of the upper surface 104 relative to the base 106, rotation of the upper surface relative to the base can also be effected. A rotation in the +a direction represents a rotational movement of the upper surface 104 about the x axis, so as to raise the head of a patient lying on the bed and to lower the feet of the patient. A movement in the -a direction represents a rotational movement of the upper surface 104 relative to the base 106 about the x axis so as to raise the feet of a patient lying on the upper surface and to lower the patient's head. Movement in the +b direction represents a rotational movement of the upper surface 104 relative to the base 106 about the y axis, and movement in the -b direction represents a rotational movement of the upper surface in a direction opposite to the +b direction. Movement in the +c direction represents a rotational movement of the upper surface 104 relative to the base 106 about the z axis, and movement in the -c direction represents a rotational movement of the upper surface about the z axis in a direction opposite to the +c direction.

Rotation in the +a and -a directions about the x axis can be considered to adjust the pitch of the upper surface 104; rotation in the +b and -b direction about the y axis can be considered to adjust the roll of the upper surface; and rotation in the +c and -c directions about the z axis can be considered to adjust to yaw of the upper surface.

Figures 2A and 2B show the control unit 200 from two different angles; Figure 2A shows a rear end 200a of the control unit in a perspective view, and Figure 2B shows a front end 200b of the control unit in a perspective view. In the described embodiment, the control unit 200 is a handheld unit intended to be held in one hand of an operator, such as technician or medical professional. The control unit 200 has a housing 202 which, in the embodiment shown, is generally rectangular cuboidal in shape. In other embodiments, however, the housing 202 may be formed in a more ergonomic shape, for example to fit comfortably into the hand of an operator.

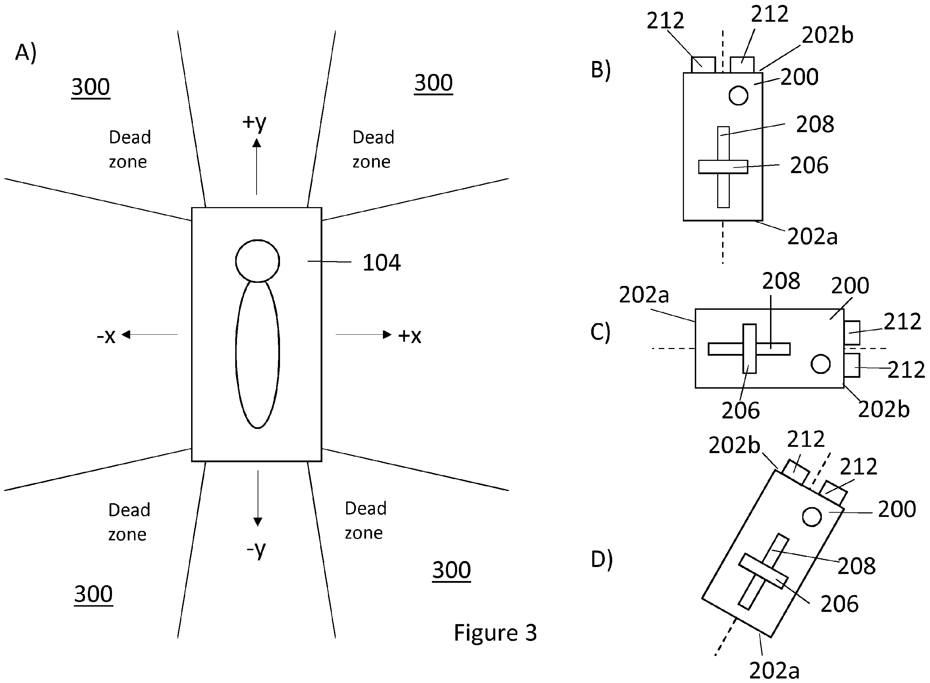

The control unit 200 has two interfaces via which a user can input commands. A first interface 204 is, in this embodiment, in the form of a slider switch which includes an actuator 206 moveable along a track 208. In one embodiment, biasing means (not shown) within the housing 202 act to urge the actuator 206 to a central position along the track 208 such that, when an operator removes his or her finger from the actuator, the actuator returns to its central position along the track, as shown in Figures 2A and 2B. An operator is able to slide the actuator 206 in either direction (that is, towards the rear end 200a or towards the front end 200b) along the track 208 in order to effect movement of the upper surface 104 of the patient support 102, as will be discussed with reference to Figures 3 to 7.

A second user interface 210, in this embodiment, takes the form of a button which functions as a toggle switch. The button 210 enables an operator to toggle between linear movement and rotational movement. That is to say, when the button 210 is pressed, the first user interface is configured to effect rotational movement of the upper surface 104 (in directions +a, -a, +b, -b, +c and -c), and when the button is not pressed, the first user interface can be used to effect linear movement of the upper surface (in directions +x, -x, +y, -y, +z and -z). Use of the control unit 200 to effect movement of the upper surface 104 will be discussed in greater detail with reference to Figures 3 to 7.

The control unit 200 may include an indicator, such as a light (not shown), to indicate to an operator when the button 210 is depressed. For example, the light may be illuminated when the button 210 is depressed and, therefore, causes the first user interface to effect rotational movement of the patient support 102.

With reference to Figure 2B, a pair of ultrasound emitters 212 are provided on the front end 200b of the control unit 200. In other embodiments, the ultrasound emitters 212 may be located elsewhere on the control unit 200. The ultrasound emitters 212 are configured to communicate with a pair of ultrasound receivers 214 (see Figure 1A) located on the base 106 of the patient 102. In some embodiments, the base 16 includes multiple pairs of ultrasound receivers 214 which are in communication with the ultrasound emitters 212. In this embodiment, a single pair of ultrasound receivers 214 is shown located on an end of the base 106. However, it will be appreciated that the ultrasound receivers 214 may, in other embodiments, be located elsewhere on the patient support 102, for example on another surface of the base or on the upper surface 104. The purpose of the ultrasound emitters 212 and the ultrasound receivers 214 is to enable a determination to be made of the approximate location of the control unit 200 relative to the patient support 102 and of the orientation of the control unit relative to the patient support.

The control unit 200 may additionally or alternatively include radio frequency identification (RFID) beacons or emitters, which can communicate with RFID trackers or receivers on the patient support 102, the base 106, or elsewhere near to the patient support, in order to determine the orientation of the control unit with respect to the base 106.

In an alternative embodiment, the control unit may include infrared emitters which can communicate with infrared receivers on the patient support 102, the base 106, or elsewhere near to the patient support, in order to determine the orientation of the control unit with respect to the base 106.

In addition to the ultrasound emitters 212 and the ultrasound receivers 214, the control unit 200 may, in some embodiments, include one or more additional sensors such as an inertial measurement device (not shown) to enable a determination to be made of the orientation of the control unit 200 relative to the patient support 102. The inertial measurement device may comprise one or more of an accelerometer, a gyroscope, a compass and a magnetometer.

It will be appreciated that, in alternative embodiments, fewer or more emitters and transmitters may be used. Thus, in the illustrated embodiment, at least one emitter/transmitter is configured to communicate with at least one receiver.

In alternative embodiments, an external sensor system (not illustrated) may be used to track one or more of the orientation and position of the control unit 200, and one or more of the orientation and position of the patient support 102. The external sensor system may then compare the position and/or orientation of the control unit 200 relative to the position and/or orientation of the patient support 102, and thus determine the direction of movement of the upper surface 104 as controlled by the control unit 200 (for which, see below).

It will be appreciated that communication between the control unit and the patient support may be achieved using a wired or cable connection (not shown).

It will be appreciated that, while emitters 212 and receivers 214 communicate with each other using ultrasound, in other embodiments, different communication modalities may be used.

Movement of the upper surface 104 of the patient support 102 using the control unit 200 will now be discussed with reference to Figures 3, 4, 5, 6 and 7.

Figures 3A to 3D show, diagrammatically, how the upper surface 104 of the patent support 102 may be moved along the x and y axes. Figure 3A shows the upper surface 104 as viewed from above. The direction of linear movement of the upper surface 104 is determined by the orientation of the control unit 200 relative to the patient support 102. The control unit 200 is considered to define a longitudinal axis and, in order for the control unit to effect linear movement of the upper surface 104 one of the x or y axes, the control unit must be oriented such that its longitudinal axis is parallel to or substantially parallel to that axis along which movement is desired. For example, to enable the control unit 200 to be used to move the upper surface 104 along the y axis, the control unit must be held in such an orientation that its longitudinal axis is parallel to or substantially parallel to the y axis, as shown in Figure 3B. The control unit is considered to be oriented substantially parallel to a particular axis if it is oriented within a defined angular range of that axis, for example within a 30 degree range. The orientation of the control unit 200 relative to the upper surface 104 is determined by the communication between the ultrasound emitters 212 on the control unit and the ultrasound receivers 214 on the base 106 of the patient support 102. For example, the time-of-flight of signals emitted from each of the emitters 212 and detected by each of the receivers 214 can be used to determine the distance of each emitter from each receiver. This information, combined with the known distances between the emitters 212 and between the receivers 214, allows the relative orientation of the control unit 200 with respect to the patient support 102 to be determined.

By including the emitters and receivers in pairs, it is possible to differentiate between the signals transmitted and received by each transmitter/receiver in a pair. The transmitters in a pair are positioned sufficiently far apart from one another that a determination can be made of how the transmitters are positioned relative to one another and, therefore, how the control unit 200 is oriented with respect to the patient support 102. Alternatively, the transmitters in a pair can communicate using different frequencies in order to be distinguished from each other, and thus determine how the control unit 200 is oriented with respect to the patient support 102. In an alternative embodiment, as is mentioned above, the orientation of the control unit 200 may additionally or alternatively be determined using one or more sensors, such as an inertial measurement device located within the housing 202 of the control unit, or RFID beacons located in the control unit and RFID trackers located on or near to the base.

With the control unit 200 oriented in the manner shown in Figure 3B (that is parallel to or substantially parallel to the y axis), a user is able to move the upper surface in the +y direction by sliding the actuator 206 along the track 208 in the +y direction (that is towards the front end 202b of the control unit), and is able to move the upper surface in the -y direction by sliding the actuator along the track in the -y direction (that is towards the rear end 202a of the control unit).

In this embodiment, the control unit 200 is oriented parallel to the y axis, with the front end 202b of the control unit aimed in the +y direction. If the control unit is held facing the opposite direction, with the front end 202b of the control unit aimed in the -y direction, then the direction of movement effected by the actuator 206 is reversed; sliding the actuator along the track 208 towards the front end of the control unit will cause the upper surface 104 to move in the -y direction. Similarly, movement of the actuator 206 along the track 208 towards the rear end 202a of the control unit 200 will cause the upper surface 104 to move in the +y direction.

In order to move the upper surface 104 along the x axis, the control unit 200 must be oriented parallel to or substantially parallel to the x axis, as shown in Figure 3C. When the control unit 200 is oriented in this way, a user is able to move the upper surface 104 of the patient support 102 in the +x direction by sliding the actuator 206 along the track 208 in the +x direction (that is towards the front end 202b of the control unit) and is able to move the upper surface in the -x direction by sliding the actuator along the track in the -x direction (that is towards the rear end 202a of the control unit).

In this embodiment, the control unit 200 is oriented parallel to the x axis, with the front end 202b of the control unit aimed in the +x direction. If the control unit is held facing the opposite direction, with the front end 202b of the control unit aimed in the -x direction, then the direction of movement effected by the actuator 206 is reversed; sliding the actuator along the track 208 towards the front end of the control unit will cause the upper surface 104 to move in the -x direction. Similarly, movement of the actuator 206 along the track 208 towards the rear end 202a of the control unit 200 will cause the upper surface 104 to move in the +x direction.

In general, therefore, if the control unit 200 is oriented parallel to or substantially parallel to an axis, then movement of the actuator 206 along the track 208 in a particular direction will cause the upper surface 104 to move in a corresponding direction along the axis.

To ensure that the upper surface 104 will only move in a particular direction when the control unit 200 is oriented substantially parallel to that direction, the patient support system 100 is configured, in one embodiment, such that the upper surface will not move if the control unit is not oriented parallel to or substantially parallel to the direction of movement. Thus, if the controller 200 is oriented such that it is not parallel to or within a defined angular range of a direction of motion (that is, if the control unit is not oriented parallel to or within a defined angular range of the x or y axes), such as in Figure 3D, then sliding the actuator 206 along the track 208 will not cause any movement of the upper surface 104. The defined angular range may be +/- 30 degrees (that is, an arc of 30 degrees either side of the axis). Preferably, the angular range will be no less than +/- 10 degrees. Regions 300 in Figure 3A denote so called "dead zones". If the control unit 200 is oriented such that its longitudinal axis is parallel to an axis within a dead zone 300, then movement of the actuator 206 along the track 208 will have no effect on the position of the upper surface 104.

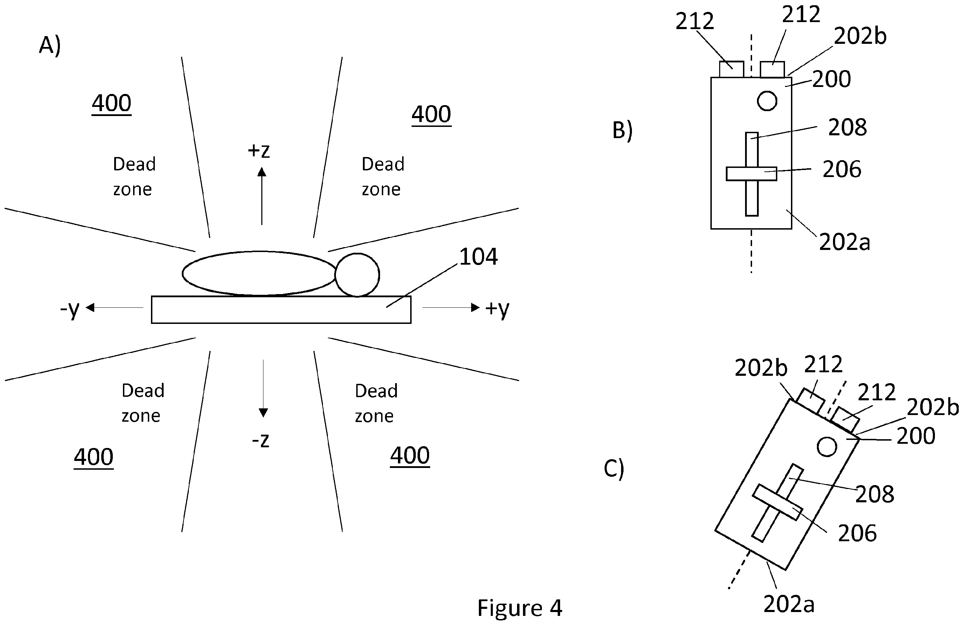

Figures 4A to D show diagrammatically how the upper surface 104 of the patent support 102 may be moved along the y and z axes. Figure 4A shows the upper surface 104 as viewed from the side. As is discussed above, in order for the control unit 200 to effect linear movement of the upper surface 104 one of the y or z axes, the control unit must be oriented such that its longitudinal axis is parallel to or substantially parallel to that axis along which movement is desired. In other words, to enable the control unit 200 to be used to move the upper surface 104 along the z axis, the control unit must be held in an orientation parallel to or substantially parallel to the z axis, as shown in Figure 4B.

With the control unit 200 oriented in the manner shown in Figure 4B (substantially parallel to the z axis), a user is able to move the upper surface in the +z direction by sliding the actuator 206 along the track 208 in the +z direction (that is towards the front end 202b of the control unit), and is able to move the upper surface in the -z direction by sliding the actuator along the track in the -z direction (that is towards the rear end 202a of the control unit).

In this embodiment, the control unit 200 is oriented parallel to the z axis, with the front end 202b of the control unit aimed in the +z direction. If the control unit is held facing the opposite direction, with the front end 202b of the control unit aimed in the -z direction, then the direction of movement effected by the actuator 206 is effectively reversed; sliding the actuator along the track 208 towards the front end of the control unit will cause the upper surface 104 to move in the -z direction. Similarly, movement of the actuator 206 along the track 208 towards the rear end 202a of the control unit 200 will cause the upper surface 104 to move in the +z direction.

Again, if the controller 200 is oriented such that it is not parallel to a direction of motion (that is, if the control unit is not oriented parallel to the y or z axis), such as in Figure 4C, then sliding the actuator 206 along the track 208 will not cause any movement of the upper surface 104. Regions 400 in Figure 4A denote so called "dead zones". If the control unit 200 is oriented such that its longitudinal axis is parallel to an axis lying within a dead zone 400, then movement of the actuator 206 along the track 208 will have no effect on the position of the upper surface 104.

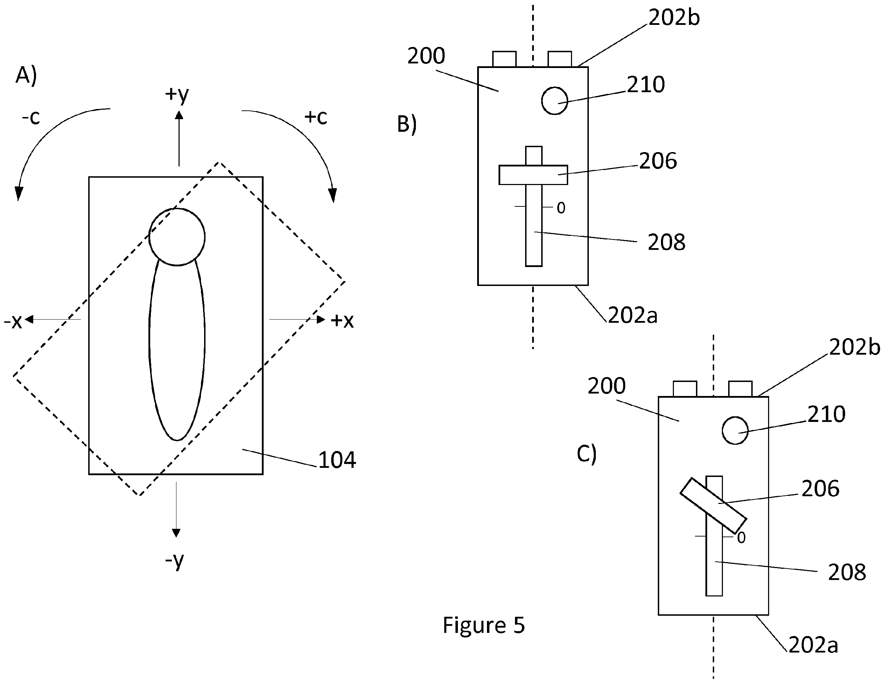

Figures 5A and 5B show how the upper surface 104 of the patient support 102 may be rotated in the +c and -c directions, about the z axis. In Figure 5A, the upper surface 104, shown in plan view, is oriented with its longitudinal axis parallel to the y axis. As is mentioned above, to effect rotational movement of the upper surface 104, the button 210 on the control unit 200 must be pressed which, effectively, puts the control unit into rotation mode. With rotation mode selected, the actuator 206 can be used to rotate the upper surface 104 in the +c and -c directions. In this embodiment, with the control unit 200 oriented as shown in Figure 5B, along the y axis, and with the front end 202b aimed in the +y direction, then sliding the actuator in the +y direction (towards the front end of the control unit, as shown in Figure 5B) causes the upper surface to rotate in the +c direction towards the position shown by a dashed line. Conversely, sliding the actuator 206 along the track 208 in the -y direction (towards the rear end 202a of the control unit 200) causes the upper surface 104 to rotate in the -c direction.

In this embodiment, orienting the control unit 200 in the opposite way, by aiming the front end 202b in the -y direction, does not change the functionality of the actuator 206; with the control unit 200 oriented in this way (that is with the front end 202b aimed in the -y direction), sliding the actuator in the -y direction (towards the front end of the control unit) will cause the upper surface 104 to rotate in the +c direction, and sliding the actuator in the +y direction (towards the rear end 202a of the control unit) will cause the upper surface to rotate in the -c direction. In other words, when the control unit is in rotation mode, and is oriented substantially parallel to the y axis, sliding the actuator 206 towards the front end 202b of the control unit causes the upper surface 104 to rotate clockwise when viewed from above (as in Figure 5A).

In an alternative embodiment, as shown in Figure 5C, the actuator 206 is rotatable with respect to the control unit 200 and/or to the track 208. For example, the actuator may be rotatable around its point of contact with the track 208. In some embodiments, this rotation may only be possible once the button 210 is depressed. In other embodiments, rotation may be possible when the button 210 is not depressed, but no rotation signal is transmitted unless the button is depressed. If the control unit is oriented substantially parallel with respect to the y axis, then rotating the actuator 206 in the +c direction will cause the upper surface 104 of the upper surface 104 of the patient support to rotate in the +c direction. Similarly, rotating the actuator in the -c direction will cause the upper surface 104 of the patient support to rotate in the -c direction. Releasing the button 210 will allow the user to return the actuator 206 to a neutral position with respect to the track 208 without moving the upper surface 104. This may be useful when further adjustments to the position of the upper surface are necessary after the upper support has been rotated.

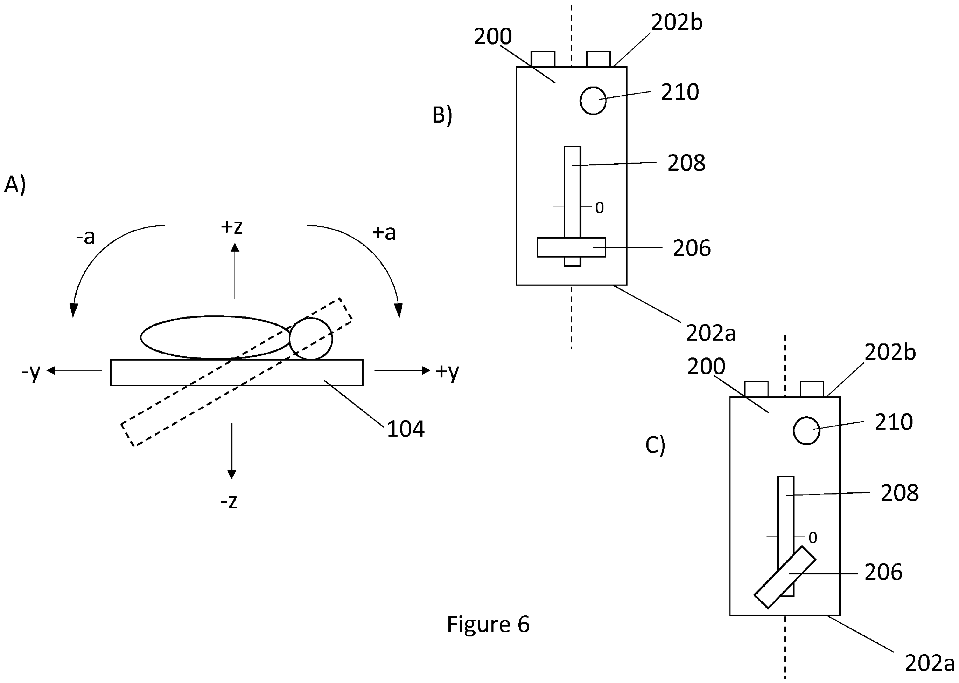

Figures 6A and 6B show the upper surface 104 of the patient support 102 may be rotated in the +a and -a directions, about the x axis. In Figure 6A, the upper surface 104, shown in a side view, is oriented with its longitudinal axis parallel to the y axis. With rotation mode selected, the actuator 206 can be used to rotate the upper surface 104 in the +a and -a directions. In this embodiment, with the control unit 200 oriented as shown in Figure 6B, along the z axis, and with the front end 202b aimed in the +z direction, then sliding the actuator in the +z direction (towards the front end of the control unit) causes the upper surface to rotate in the +a direction. Conversely, sliding the actuator 206 along the track 208 in the -z direction (towards the rear end 202a of the control unit 200, as shown in Figure 6B) causes the upper surface 104 to rotate in the -a direction, towards the position shown by the dashed line in Figure 6A.

In this embodiment, orienting the control unit 200 in the opposite way, by aiming the front end 202b in the -z direction, does not change the functionality of the actuator 206; with the control unit 200 oriented in this way (that is with the front end 202b aimed in the -z direction), sliding the actuator in the -z direction (towards the front end of the control unit) will cause the upper surface 104 to rotate in the +a direction, and sliding the actuator in the +z direction (towards the rear end 202a of the control unit) will cause the upper surface to rotate in the -a direction. In other words, when the control unit is in rotation mode, and is oriented substantially parallel to the z axis, sliding the actuator 206 towards the front end 202b of the control unit causes the upper surface 104 to rotate clockwise when viewed from the side (as in Figure 6A).

In an alternative embodiment, as shown in Figure 6C, the actuator 206 is rotatable with respect to the control unit 200 and/or to the track 208. If the control unit is oriented substantially parallel with respect to the z axis, then rotating the actuator 206 in the +a direction will cause the upper surface 104 of the upper surface 104 of the patient support to rotate in the +a direction. Similarly, rotating the actuator in the -a direction will cause the upper surface 104 of the patient support to rotate in the -a direction.

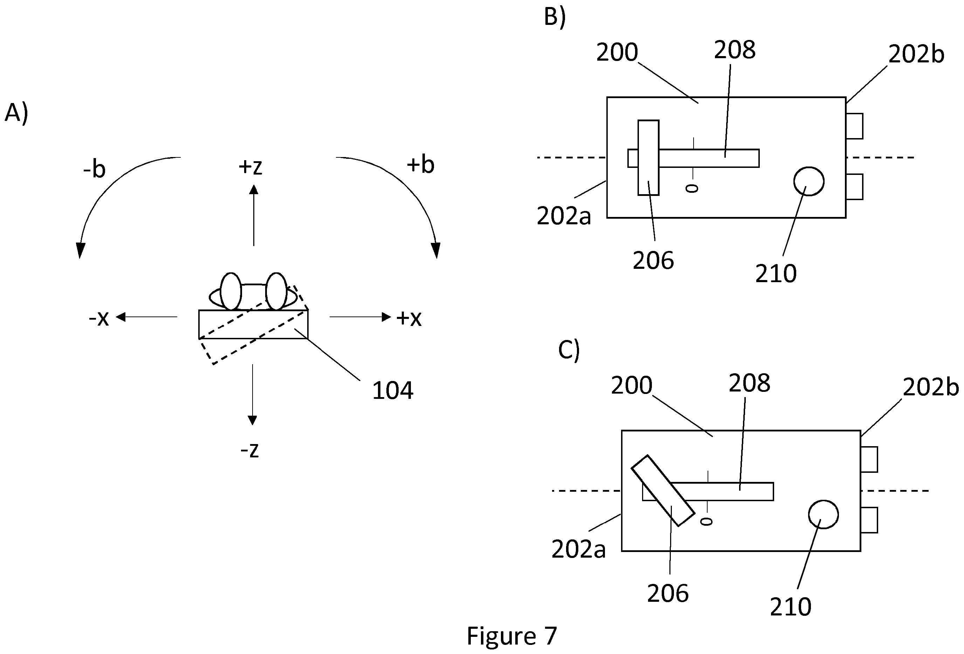

Figures 7A and 7B show how the upper surface 104 of the patient support 102 may be rotated in the +b and -b directions, about the y axis. In Figure 7A, the upper surface 104, shown in an end view, is oriented with its longitudinal axis parallel to the y axis. With rotation mode selected, the actuator 206 can be used to rotate the upper surface 104 in the +b and -b directions. In this embodiment, with the control unit 200 oriented as shown in Figure 7B, along the x axis, and with the front end 202b aimed in the +x direction, then sliding the actuator in the +x direction (towards the front end of the control unit) causes the upper surface to rotate in the +b direction. Conversely, sliding the actuator 206 along the track 208 in the -x direction (towards the rear end 202a of the control unit 200, as shown in Figure 7B) causes the upper surface 104 to rotate in the -b direction, towards the position shown by the dashed line in Figure 7A.

In this embodiment, orienting the control unit 200 in the opposite way, by aiming the front end 202b in the -x direction, does not change the functionality of the actuator 206; with the control unit 200 oriented in this way (that is with the front end 202b aimed in the -x direction), sliding the actuator in the -x direction (towards the front end of the control unit) will cause the upper surface 104 to rotate in the +b direction, and sliding the actuator in the +x direction (towards the rear end 202a of the control unit) will cause the upper surface to rotate in the -b direction. In other words, when the control unit is in rotation mode, and is oriented substantially parallel to the x axis, sliding the actuator 206 towards the front end 202b of the control unit causes the upper surface 104 to rotate clockwise when viewed from the end (as in Figure 7A).

While, in the embodiments described above, orienting the control unit 200 in a particular way with respect to the patient support 102 causes the upper surface 104 to move in a particular direction, those directions, in other embodiments, may be reversed or varied. For example, orienting the control unit 200 such that it is parallel to the y axis may cause movement of the upper surface 104 along the x or z axes.

In an alternative embodiment, as shown in Figure 7C, the actuator 206 is rotatable with respect to the control unit 200 and/or to the track 208. If the control unit is oriented substantially parallel with respect to the x axis, then rotating the actuator 206 in the +b direction will cause the upper surface 104 of the upper surface 104 of the patient support to rotate in the +b direction. Similarly, rotating the actuator in the -b direction will cause the upper surface 104 of the patient support to rotate in the -b direction.

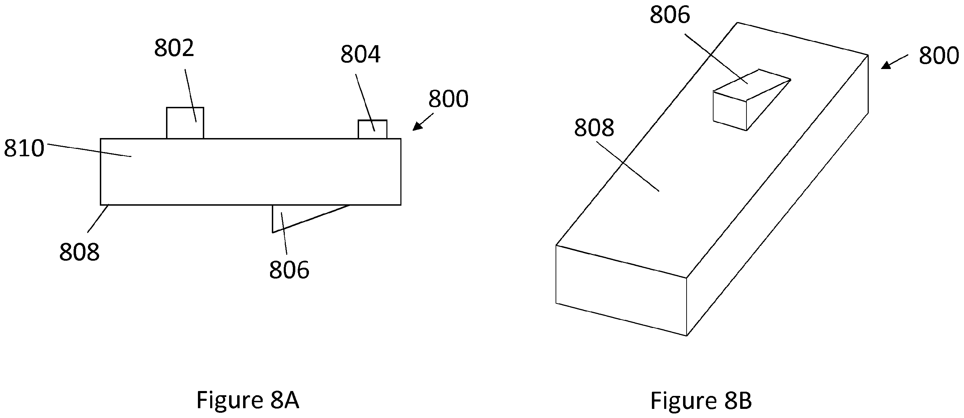

Figures 8A and 8B show a control unit 800 constructed in accordance with an alternative embodiment of the invention. Figure 8A is a side view of the control unit 800 which includes an actuator 802 and a button 804, which correspond to the actuator 206 and the button 210 of the control unit 200 of Figures 2A and 2B. In this embodiment, however, the control unit 800 includes a spring-loaded switch 806 extending from a bottom wall 808 of a housing 810 of the control unit. The switch 806 functions as a "dead man's switch" so that functionality of the actuator 802 and the button 804 is only enabled if the switch 806 is pressed. The location of the switch 806 enables a user to press the switch with his or her finger while holding the control unit 800. Thus, a user holding the control unit 800 is able to press the switch 806 with his or her finger and, simultaneously, control the actuator 802 and the button 804 with his or her thumb. The switch 806 is spring-loaded such that, if the user removes his or her finger from the switch, for example if the user drops the control unit 800, and pressure on the switch is removed, then the switch rapidly opens, thereby preventing further movement of the upper surface 104 of the patient support 102.

So far, the invention has been described in terms of individual embodiments. However, those skilled in the art will appreciate that various embodiments of the invention, or features from one or more embodiments, may be combined as required. It will be appreciated that various modifications may be made to these embodiments without departing from the scope of the invention, which is defined by the appended claims.

## Claims

1. A patient support system (100), comprising:
a patient support (102) having a base (106) and an upper surface (104), the upper surface moveable relative to the base in a direction along at least one of a first axis, a second axis orthogonal to the first axis, and a third axis orthogonal to the first and second axes; and
a control unit (200) for controlling movement of the upper surface, the control unit defining a longitudinal axis;
means for determining the orientation of the control unit (200) with respect to the patient support (102);
wherein the direction of movement of the upper surface relative to the base is determined by the orientation of the longitudinal axis of the control unit relative to the patient support;
wherein, when the control unit (200) is oriented such that its longitudinal axis is parallel to or within a defined angular range of the first axis, the upper surface (104) is moveable along the first axis, when the control unit is oriented such that its longitudinal axis is parallel to or within a defined angular range of the second axis, the upper surface is moveable along the second axis, and when the control unit is oriented such that its longitudinal axis is parallel to or within a defined angular range of the third axis, the upper surface is moveable along the third axis; and
wherein, when the control unit (200) is oriented such that its longitudinal axis is outside any of the defined angular ranges, control of the movement of the upper surface (104) by the control unit is prohibited.

2. A patient support system (100) according to claim 1, wherein the defined angular range about each axis is a range of 30 degrees.

3. A patient support system (100) according to any of the preceding claims, wherein the control unit (200) comprises a first user interface (204) for receiving a user input, and wherein the upper surface (104) is configured to move in response to a user input received via the first user interface.

4. A patient support system (100) according to claim 3, wherein the first user interface (204) comprises a sliding input device, the sliding input device (206) moveable along a track (208).

5. A patient support system (100) according to claim 4, wherein the control unit (200) has a front end (200b) and a rear end (200a), and the sliding input device (206) is movable between a front end of the track (208) and a rear end of the track, the front end and the rear end of the track corresponding to the front end and the rear end of the control unit respectively.

6. A patient support system (100) according to claim 5, wherein, when the control unit (200) is oriented such that its longitudinal axis is parallel to or within the defined angular range of a particular one of the first, second and third axes, the direction of movement of the upper surface (104) along the particular axis corresponds to a direction of movement of the sliding input device (206) along the track (208).

7. A patient support system (100) according to any of claims 4 to 6, wherein the sliding input device (206) is rotatable with respect to the control unit (200).

8. A patient support system (100) according to any of the preceding claims, wherein the upper surface (104) is rotationally moveable relative to the base (106) about at least one of the first axis, the second axis, and the third axis.

9. A patient support system (100) according to claim 8, wherein the control unit (200) further comprises a toggle switch (210), moveable between a first position and a second position, and wherein, when the toggle switch is in the first position, the first user interface is configured to effect linear movement of the upper surface (104) and, when the toggle switch is in the second position, the first user interface is configured to effect rotational movement of the upper surface.

10. A patient support system (100) according to claim 8 or claim 9, wherein, when the control unit (200) is oriented such that its longitudinal axis is parallel to or within the defined angular range of the first axis, the upper surface (104) is rotatable about one of the first, second or third axes, when the control unit is oriented such that its longitudinal axis is parallel to or within the defined angular range of the second axis, the upper surface is rotatable about another of the first, second or third axes, and when the control unit is oriented such that its longitudinal axis is parallel to or within the defined angular range of the third axis, the upper surface is rotatable about the other of the first, second or third axes.

11. A patient support system (100) according to any of the preceding claims, wherein the control unit (200) includes at least one transmitter (212) and the patient support includes at least one receiver (214), and wherein the at least one transmitter is configured to communicate with the at least one receiver in order for the orientation of the control unit relative to the patient support to be determined.

12. A patient support system (100) according to claim 11, wherein the at least one transmitter comprises an ultrasound transmitter and the at least one receiver comprises an ultrasound receiver, or the at least one transmitter comprises an infrared transmitter and the at least one receiver comprises an infrared receiver, or the at least one transmitter comprises an RFID transmitter and the at least one receiver comprises an RFID receiver.

13. A patient support system (100) according to any of claims 1 to 10, wherein the control unit (200) comprises an inertial measurement device for determining the orientation of the control unit relative to the patient support.

## Patentansprüche

1. Patientenstützsystem (100), umfassend:
eine Patientenstütze (102) mit einer Basis (106) und einer oberen Fläche (104), wobei die obere Fläche relativ zur Basis in einer Richtung entlang einer ersten Achse oder einer zweiten Achse orthogonal zur ersten Achse und einer dritten Achse orthogonal zur ersten und zweiten Achse beweglich ist; und
eine Steuereinheit (200) zum Steuern der Bewegung der oberen Fläche, wobei die Steuereinheit eine Längsachse definiert;
Mittel zum Bestimmen der Ausrichtung der Steuereinheit (200) in Bezug auf die Patientenstütze (102);
wobei die Bewegungsrichtung der oberen Fläche relativ zur Basis durch die Ausrichtung der Längsachse der Steuereinheit relativ zur Patientenstütze bestimmt wird;
wobei die obere Fläche (104) entlang der ersten Achse bewegt werden kann, wenn die Steuereinheit (200) so ausgerichtet ist, dass ihre Längsachse parallel zur ersten Achse oder in einem definierten Winkelbereich der ersten Achse verläuft, wobei die obere Fläche entlang der zweiten Achse bewegt werden kann, wenn die Steuereinheit so ausgerichtet ist, dass ihre Längsachse parallel zur zweiten Achse oder in einem definierten Winkelbereich der zweiten Achse verläuft und wobei die obere Fläche entlang der dritten Achse bewegt werden kann, wenn die Steuereinheit so ausgerichtet ist, dass ihre Längsachse parallel zur dritten Achse oder in einem definierten Winkelbereich der dritten Achse verläuft; und
wobei die Steuereinheit die Steuerung der Bewegung der oberen Fläche (104) verhindert, wenn die Steuereinheit (200) so ausgerichtet ist, dass ihre Längsachse außerhalb des definierten Winkelbereichs verläuft.

2. Patientenstützsystem (100) gemäß Anspruch 1, wobei der definierte Winkelbereich um jede Achse in einem Bereich von 30 Grad liegt.

3. Patientenstützsystem (100) gemäß einem der vorhergehenden Ansprüche, wobei die Steuereinheit (200) eine erste Benutzerschnittstelle (204) zum Empfangen einer Benutzereingabe umfasst und wobei die obere Oberfläche (104) dafür ausgelegt ist, sich als Reaktion auf eine über die erste Benutzerschnittstelle empfangene Benutzereingabe zu bewegen.

4. Patientenstützsystem (100) gemäß Anspruch 3, wobei die erste Benutzerschnittstelle (204) eine Schiebeeingabevorrichtung umfasst und die Schiebereingabevorrichtung (206) entlang einer Spur (208) bewegt werden kann.

5. Patientenstützsystem (100) gemäß Anspruch 4, wobei die Steuereinheit (200) ein vorderes Ende (200b) und ein hinteres Ende (200a) aufweist und die Schiebeeingabevorrichtung (206) zwischen einem vorderen Ende der Spur (208) und einem hinteren Ende der Spur bewegt werden kann und wobei das vordere Ende und das hintere Ende der Spur jeweils dem vorderen Ende und dem hinteren Ende der Steuereinheit entsprechen.

6. Patientenstützsystem (100) gemäß Anspruch 5, wobei die Bewegungsrichtung der oberen Fläche (104) entlang einer bestimmten Achse der Bewegungsrichtung der Schiebeeingabevorrichtung (206) entlang der Spur (208) entspricht, wenn die Steuereinheit (200) so ausgerichtet ist, dass ihre Längsachse parallel zur ersten, zweiten oder dritten Achse oder innerhalb des definierten Winkelbereichs der ersten, zweiten oder dritten Achse verläuft.

7. Patientenstützsystem (100) gemäß einem der Ansprüche 4 bis 6, wobei die Schiebeingabevorrichtung (206) in Bezug auf die Steuereinheit (200) gedreht werden kann.

8. Patientenstützsystem (100) gemäß einem der vorhergehenden Ansprüche, wobei die obere Fläche (104) relativ zur Basis (106) um die erste Achse, die zweite Achse oder um die dritte Achse gedreht werden kann.

9. Patientenstützsystem (100) gemäß Anspruch 8, wobei die Steuereinheit (200) ferner einen zwischen einer ersten Position und einer zweiten Position bewegbaren Kippschalter (210) umfasst und wobei die erste Benutzerschnittstelle dafür ausgelegt ist, eine lineare Bewegung der oberen Fläche (104) zu bewirken, wenn sich der Kippschalter in der ersten Position befindet und die erste Benutzerschnittstelle dafür ausgelegt ist, eine Drehbewegung der oberen Fläche zu bewirken, wenn sich der Kippschalter in der zweiten Position befindet.

10. Patientenstützsystem (100) gemäß Anspruch 8 oder Anspruch 9, wobei die obere Fläche (104) um die erste, zweite oder dritte Achse gedreht werden kann, wenn die Steuereinheit (200) so ausgerichtet ist, dass ihre Längsachse parallel zur ersten Achse oder in einem definierten Winkelbereich der ersten Achse verläuft, wobei die obere Fläche um die erste, zweite oder dritte Achse gedreht werden kann, wenn die Steuereinheit so ausgerichtet ist, dass ihre Längsachse parallel zur zweiten Achse oder in einem definierten Winkelbereich der zweiten Achse verläuft und wobei die obere Fläche um die erste, zweite oder dritte Achse gedreht werden kann, wenn die Steuereinheit so ausgerichtet ist, dass ihre Längsachse parallel zur dritten Achse oder in einem definierten Winkelbereich der dritten Achse verläuft.

11. Patientenstützsystem (100) gemäß einem der vorhergehenden Ansprüche, wobei die Steuereinheit (200) mindestens einen Sender (212) umfasst und die Patientenstütze mindestens einen Empfänger (214) umfasst und wobei mindestens ein Sender dafür ausgelegt ist, mit mindestens einem Empfänger zu kommunizieren, um die Ausrichtung der Steuereinheit relativ zur Patientenstütze zu bestimmen.

12. Patientenstützsystem (100) gemäß Anspruch 11, wobei mindestens ein Sender einen Ultraschallsender umfasst und mindesten ein Empfänger einen Ultraschallempfänger umfasst oder mindestens ein Sender einen Infrarotsender umfasst und mindestens ein Empfänger einen Infrarotempfänger umfasst oder mindestens ein Sender einen RFID-Sender umfasst und mindestens ein Empfänger einen RFID-Empfänger umfasst.

13. Patientenstützsystem (100) gemäß einem der Ansprüche 1 bis 10, wobei die Steuereinheit (200) eine IMU zur Bestimmung der Ausrichtung der Steuereinheit relativ zur Patientenstütze umfasst.

## Revendications

1. Un système de support de patient (100), comprenant :
un support de patient (102) ayant une base (106) et une surface supérieure (104), la surface supérieure étant mobile par rapport à la base dans une direction le long d'au moins l'un d'un premier axe, un second axe orthogonal au premier axe, et un troisième axe orthogonal aux premier et deuxième axes ; et une unité de commande (200) pour commander le mouvement de la surface supérieure, l'unité de commande définissant un axe longitudinal ;
des moyens pour déterminer l'orientation de l'unité de commande (200) par rapport au support de patient (102) ;
dans lequel la direction du mouvement de la surface supérieure par rapport à la base est déterminée par l'orientation de l'axe longitudinal de l'unité de commande par rapport au support de patient ;
dans lequel, lorsque l'unité de commande (200) est orientée de sorte que son axe longitudinal est parallèle ou dans une gamme angulaire définie du premier axe, la surface supérieure (104) est mobile le long du premier axe, lorsque l'unité de commande est orientée que son axe longitudinal est parallèle ou à l'intérieur d'une gamme angulaire définie du deuxième axe, la surface supérieure est mobile le long du second axe et lorsque l'unité de commande est orientée de telle sorte que son axe longitudinal soit parallèle ou dans une gamme angulaire définie du troisième axe, la surface supérieure est mobile le long du troisième axe ; et
dans lequel, lorsque l'unité de commande (200) est orientée de sorte que son axe longitudinal est à l'extérieur de l'une quelconque des gammes angulaires définies, le contrôle du mouvement de la surface supérieure (104) par l'unité de commande est interdit.

2. Un système de support de patient (100) selon la revendication 1, dans lequel la gamme angulaire définie autour de chaque axe est une gamme de 30 degrés.

3. Un système de support de patient (100) selon l'une quelconque des revendications précédentes, dans lequel l'unité de commande (200) comprend une première interface d'utilisateur (204) pour recevoir une entrée de l'utilisateur, et dans lequel la surface supérieure (104) est configurée pour se déplacer en réponse à une entrée d'utilisateur reçue via la première interface d'utilisateur.

4. Un système de support de patient (100) selon la revendication 3, dans lequel la première interface d'utilisateur (204) comprend un dispositif d'entrée coulissant, le dispositif d'entrée coulissant (206) pouvant être déplacé le long d'une piste (208).

5. Un système de support de patient (100) selon la revendication 4, dans lequel l'unité de commande (200) a une extrémité avant (200b) et une extrémité arrière (200a), et le dispositif d'entrée coulissant (206) est mobile entre une extrémité avant de la piste (208) et une extrémité arrière de la piste, l'extrémité avant et l'extrémité arrière de la piste correspondant respectivement à l'extrémité avant et à l'extrémité arrière de l'unité de commande.

6. Un système de support de patient (100) selon la revendication 5, dans lequel, lorsque l'unité de commande (200) est orientée de telle sorte que son axe longitudinal est parallèle ou dans la gamme angulaire définie d'un premier, deuxième et troisième axes, la direction de déplacement de la surface supérieure (104) le long de l'axe particulier correspond à une direction de déplacement du dispositif d'entrée coulissant (206) le long de la piste (208).

7. Un système de support de patient (100) selon l'une quelconque des revendications 4 à 6, dans lequel le dispositif d'entrée coulissant (206) peut tourner par rapport à l'unité de commande (200).

8. Un système de support de patient (100) selon l'une quelconque des revendications précédentes, dans lequel la surface supérieure (104) est mobile en rotation par rapport à la base (106) autour d'au moins un du premier axe, du second axe et du troisième axe.

9. Un système de support de patient (100) selon la revendication 8, dans lequel l'unité de commande (200) comprend en outre un interrupteur à bascule (210), mobile entre une première position et une seconde position, et dans lequel, lorsque l'interrupteur à bascule est dans la première position, la première interface d'utilisateur est configurée pour effectuer un mouvement linéaire de la surface supérieure (104) et, lorsque l'interrupteur à bascule est dans la seconde position, la première interface d'utilisateur est configurée pour effectuer un mouvement de rotation de la surface supérieure.

10. Un système de support de patient (100) selon la revendication 8 ou la revendication 9, dans lequel, lorsque l'unité de commande (200) est orientée de telle sorte que son axe longitudinal est parallèle ou dans la gamme angulaire définie du premier axe, la surface supérieure (104) peut tourner autour de l'un des premier, deuxième ou troisième axes, lorsque l'unité de commande est orientée de telle sorte que son axe longitudinal est parallèle ou dans la gamme angulaire définie du deuxième axe, la surface supérieure peut tourner autour d'un autre premier, deuxième ou troisième axes, et lorsque l'unité de commande est orientée de sorte que son axe longitudinal soit parallèle à ou dans la gamme angulaire définie du troisième axe, la surface supérieure peut tourner autour de l'autre des premier, deuxième ou troisième axes.

11. Un système de support de patient (100) selon l'une quelconque des revendications précédentes, dans lequel l'unité de commande (200) comprend au moins un émetteur (212) et le support de patient comprend au moins un récepteur (214), et dans lequel au moins un émetteur est configuré pour communiquer avec le au moins un récepteur afin que l'orientation de l'unité de commande par rapport au support du patient soit déterminée.

12. Un système de support de patient (100) selon la revendication 11, dans lequel le au moins un émetteur comprend un émetteur à ultrasons et au moins un récepteur comprend un récepteur à ultrasons, ou au moins un émetteur comprend un émetteur infrarouge et au moins un récepteur comprend un récepteur infrarouge, ou au moins un émetteur comprend un émetteur RFID et au moins un récepteur comprend un récepteur RFID.

13. Un système de support de patient (100) selon l'une quelconque des revendications de 1 à 10, dans lequel l'unité de commande (200) comprend un dispositif de mesure inertiel pour déterminer l'orientation de l'unité de commande par rapport au support du patient.
